Europäisches Patentamt

European Patent Office

Office européen des brevets

(19)

(11) Numéro de publication : **0 631 773 A1**

## (12) DEMANDE DE BREVET EUROPEEN

(21) Numéro de dépôt : **94401459.6**

(51) Int. Cl.⁶ : **A61K 7/48,** C12N 5/08

(22) Date de dépôt : **28.06.94**

(30) Priorité : **02.07.93 FR 9308110**

(43) Date de publication de la demande :
**04.01.95 Bulletin 95/01**

(84) Etats contractants désignés :
**AT BE CH DE DK ES FR GB GR IE IT LI LU NL
PT SE**

(71) Demandeur : **ROUSSEL-UCLAF
35, Boulevard des Invalides
F-75007 Paris (FR)**

(72) Inventeur : **Spanoli, Roberto
9, rue Brémontier
F-75017 Paris (FR)**
Inventeur : **Varkados, Margaret
11, rue Pérignon
F-75015 Paris (FR)**

(74) Mandataire : **Vieillefosse, Jean-Claude et al
ROUSSEL UCLAF,
111 Route de Noisy
F-93235 Romainville Cédex (FR)**

(54) **Nouvelles compositions cosmétiques ou dermatologiques renfermant des ribosomes, préparation des ribosomes, préparation desdites compositions et leur application.**

(57) Compositions cosmétiques ou dermatologiques pour les soins de la peau, caractérisées en ce qu'elles renferment des ribosomes extraits de cellules végétales, animales ou de micro-organismes.

EP 0 631 773 A1

La présente invention concerne de nouvelles compositions cosmétiques ou dermatologiques pour les soins de la peau, caractérisées en ce qu'elles renferment des ribosomes extraits de cellules végétales, animales ou de micro-organismes.

Ces nouvelles compositions sont destinées notamment à retarder les signes de vieillissement de la peau.

Au fur et à mesure du développement du vieillissement cutané, des modifications interviennent, surtout au niveau épidermique et dermique.

Au sein de l'épiderme, la production de cellules nouvelles ne compense plus la desquamation et l'épiderme s'amincit progressivement.

Les glandes sébacées sont fonctionnellement moins actives et la peau devient alors sèche.

Au niveau du derme, la formation de collagène jeune, responsable de la tonicité cutanée, se ralentit par diminution de l'activité secrétrice des fibroblastes. Des liaisons croisées intermoléculaires au sein des fibres de collagène se multiplient, entraînant une rigidité structurale, une réduction de la capacité d'absorption d'eau, une diminution des apports nutritionnels et d'oxygène.

Ces transformations néfastes provoquent une perte d'élasticité, une déshydratation, une asphyxie et une sécheresse cutanée.

Ces phénomènes conduisent à l'apparition de rides, notamment au niveau du visage où la peau est particulièrement agressée par des facteurs d'origine externe (intempéries, pollution, radiations lumineuses) et par des facteurs d'origine interne (maladies, augmentation de l'âge).

De nombreuses préparations cosmétiques destinées à combattre le vieillissement de la peau existent déjà sur le marché.

Ces préparations connues renferment des composés très variés, tels que des extraits biologiques, par exemple des extraits placentaires, du collagène, des mélanges polyvitaminés, des acides gras essentiels.

On connait également des compositions cosmétiques renfermant du sérum de veau foetal, des extraits d'organes tels que thymus ou rate, des extraits animaux, décrites par exemple dans la demande de brevet français n° 84 19446.

Cependant jamais encore n'a été décrite l'utilisation en cosmétologie des propriétés topiques réelles de ribosomes, ni n'ont été utilisés les ribosomes extraits de cellules végétales, animales ou de micro-organismes, pour lutter contre le vieillissement cutané.

Les ribosomes sont des organelles sub-cellulaires composés d'ARN et de protéines impliqués dans la synthèse des protéines.

A titre d'illustrations, on peut noter que les ribosomes de E. coli (gram⁻) sont constitués des fractions :

70 S     (M : $2,7.10^6$ dalton)
50 S     (M : $1,8.10^6$ dalton)
30 S     (M : $0,9.10^6$ dalton)

A leur tour les fractions 50 S et 30 S ont la composition suivante :

50 S :     34 protéines, 2ARN (23 S, 5S)
30 S :     21 protéines, 1ARN (16 S).

Il vient d'être démontré et c'est l'objet de la présente invention que ces ribosomes possèdent un remarquable pouvoir stimulant et régénérant et qu'ils sont doués d'un effet stimulant sur la croissance cellulaire.

Ces différentes propriétés illustrées plus loin dans la partie expérimentale, peuvent être avantageusement mises à profit en dermatologie et en cosmétologie.

L'un des avantages qu'apportent les compositions de la présente invention sur les compositions connues de l'art antérieur est, par exemple en remplaçant le sérum de veau foetal par des ribosomes extraits de cellules végétales, animales ou de micro-organismes, d'éviter les risques d'infection virale, tels que par exemple les risques d'infection par des prions qui sont de nouveaux agents infectieux apparus depuis le milieu des années 1980 et responsables de l'épidémie dite des vaches folles ou encéphalite bovine spongiforme.

D'autre part, l'identité des souches ou, en général, des cellules, à partir desquelles on extrait les ribosomes est facile à vérifier en permanence, ce qui assure une parfaite reproductibilité de l'extrait employé quant à son origine génétique et à sa composition.

La présente invention concerne notamment des compositions cosmétiques ou dermatologiques telles que définies ci-dessus, caractérisées en ce que les ribosomes sont extraits de micro-organismes eucaryotes ou procaryotes, de bactéries Gram⁻ choisies dans le groupe formé par Klebsiella pneumoniae, Hafnia, Escherichia coli, Klebsiella ozoenae, Enterobacter chloacae, Pseudomonas aeruginosa et Proteus ou de bactéries Gram⁺ telles que par exemple le Bacillus subtilis, Arthrobacter sp., Lactobacillus ou le Staphylococcus tel que Staphylococcus aureus.

Parmi les ribosomes extraits de bactéries gram⁻ on retient notamment les ribosomes extraits des bactéries gram⁻ choisies dans le groupe formé par Klebsiella pneumoniae, Hafnia ou Escherichia coli.

La présente invention concerne donc plus particulièrement les compositions telles que définies ci-dessus, caractérisées en ce que les ribosomes sont extraits de bactéries Gram⁻ choisies dans le groupe formé par Klebsiella pneumoniae, Hafnia, Escherichia coli.

Parmi les ribosomes extraits de Klebsiella pneumoniae on peut citer par exemple les ribosomes extraits de la souche de Klebsiella pneumoniae déposée à l'Institut Pasteur à Paris sous le n° I-163.

Parmi les ribosomes extraits d'Hafnia, on peut citer par exemple les ribosomes extraits de la souche d'Hafnia déposée à l'Institut Pasteur à Paris sous le n° I-868.

Parmi les ribosomes extraits d'Escherichia coli, on peut citer par exemple les ribosomes extraits de la souche d'Escherichia coli, déposée à l'Institut Pasteur à Paris sous le n° I-870 ou de la souche MRE600 du Microbiological Research Establishment, Salisbury, England.

Dans les compositions de l'invention, l'extrait de ribosomes peut être utilisé sous forme pulvérulente ou sous forme de suspension aqueuse.

L'invention concerne notamment des compositions cosmétiques ou dermatologiques telles que définies ci-dessus, caractérisées en ce qu'elles renferment $5.10^{-5}$ % à 1 % en poids d'une suspension de ribosomes extraits de micro-organismes ou de cellules d'organisme supérieur : cellules végétales ou animales.

L'invention concerne plus particulièrement les compositions telles que définies ci-dessus, caractérisées en ce qu'elles renferment 0,005 % à 0,05 % en poids d'une suspension de ribosomes extraits de cellules végétales, animales et notamment de micro-organismes.

L'invention concerne tout particulièrement les compositions telles que définies ci-dessus, caractérisées en ce qu'elles renferment 0,01 % en poids d'une suspension de ribosomes extraits de cellules végétales, animales et notamment de micro-organismes.

Les ribosomes peuvent être préparés selon les méthodes usuelles connues de l'homme du métier.

La présente invention s'est également attachée à la préparation des ribosomes extraits de cellules végétales, animales et notamment de micro-organismes.

La présente invention concerne donc également les compositions telles que définies ci-dessus, caractérisées en ce que les ribosomes extraits de micro-organismes sont préparés selon les étapes successives suivantes :
- culture sur un milieu liquide ou solide de la souche de micro-organismes,
- récolte après complet développement des micro-organismes,
- lyse des micro-organismes,
  et traitement de ce lysat par centrifugation, précipitation au sulfate d'ammonium, centrifugation, tamisage moléculaire, précipitation au PEG 6000, centrifugation et dialyse de la solution obtenue.

Les milieux sur lesquels peuvent être cultivées les micro-organismes sont les milieux liquide ou solide usuels et bon marché incluant une source de carbone, une source d'azote d'origine naturelle ou inorganique et des sels minéraux, tels qu'ils sont bien connus par l'homme du métier. La littérature donne de nombreux exemples de tels milieux de culture. On peut citer par exemple le chapitre 1.6 "Culture media" du livre "Biochemical Engineering and Biotechnology Handbook", 2nde édition 1991 (Stockton Press).

Les micro-organismes après récolte sont repris dans un tampon tel que par exemple le tampon polymix I :

| MgCl$_2$ | 5 mM | pH = 7,5 |
|---|---|---|
| CaCl$_2$ | 0,5 mM | |
| Putrescine | 8 mM | |
| Spermidine | 1 mM | |
| Tricine | 20 mM | |
| NH$_4$Cl | 5 mM | |
| KCl | 95 mM | |
| DTT | 1 mM | |
| ou encore dans le tampon : | | |
| Tris/Hcl | 20 mM | pH = 7,6 |
| NH$_4$Cl | 100 mM | |
| Mg(OAc)$_2$ | 10 mM | |

On procède alors à la lyse des micro-organismes par exemple par lyse à haute pression ou encore par broyage à l'alumine.

Puis on centrifuge le lysat obtenu à environ 10.000 à 13.000 rpm et précipite les protéines de préférence au sulfate d'ammonium, à la concentration de 210 mg/ml et ajusté à pH = 7,5. Après une nouvelle centrifugation, puis un tamisage moléculaire et une élution dans le tampon polymix II :

| MgCl$_2$ | 5 mM | pH = 7,5 |
|---|---|---|
| CaCl$_2$ | 0,5 mM | |
| Putrescine | 8 mM | |
| Spermidine | 1 mM | |
| Tricine | 20 mM | |
| NH$_4$Cl | 150 mM | |
| KCl | 95 mM | |
| DTT | 1 mM | |

la fraction contenant les ribosomes est précipitée de préférence par du PEG 6000 à 100 mg/ml.

Après centrifugation, la solution obtenue est dialysée environ une nuit contre un tampon tel que par exemple

| Tris/Hcl | 20 mM | |
|---|---|---|
| NH$_4$Cl | 60 mM | |
| Mg(OAc)$_2$ | 10 mM | pH = 7,6 |

On obtient ainsi les ribosomes extraits de micro-organismes attendus.

Un tel exemple de préparation des ribosomes est donné ci-après.

On peut noter que le protocole de purification des ribosomes pourrait être arrêté soit à l'issue des centrifugations après lyse des cellules des micro-organismes, soit à l'issue de la centrifugation après l'étape de précipitation des protéines au sulfate d'ammonium.

Jusqu'ici il était connu et usuel d'utiliser en culture cellulaire in vitro comme facteur de croissance cellulaire des composés en mélange tels que du sérum animal, en concentration de l'ordre de 10 %, et plus particulièrement le sérum de veau foetal, ou des substituants synthétiques de celui-ci qui sont commercialisés à un prix élevé et dont la composition est tenue secrète, ex. Ultroser HY (IBF), NU-SERUM (Sochibo), et/ou des facteurs individuels tel le facteur de croissance épidermique "EGF" (Epidermal Growth Factor).

Jamais encore, et c'est également l'objet de la présente invention, n'avaient été utilisés des ribosomes comme facteur de croissance cellulaire in vitro, à des concentrations, en plus, très faibles qui ont été définies ci-dessus.

La présente invention concerne donc particulièrement l'utilisation en alternative économiquement très avantageuse aux suppléments ci-dessus cités, de ribosomes extraits de micro-organismes comme facteur de croissance de cultures cellulaires in vitro.

De tels ribosomes peuvent être préparés ainsi qu'il est indiqué ci-dessus.

Dans le cadre de la présente invention, on a étudié le pouvoir stimulant des ribosomes selon l'invention par comparaison avec celui du sérum de veau foetal et du facteur de croissance épidermique (EGF) constituant ici le milieu témoin positif, dans des cultures de kératinocytes de l'épiderme humain.

Un exemple d'une telle étude, qui montre la supériorité i du pouvoir stimulant sur la croissance cellulaire des ribosomes sur les composés connus et couramment utilisés qui sont le sérum de veau foetal et le facteur de croissance épidermique EGF est décrit ci-après.

La présente invention concerne donc tout particulièrement l'utilisation de ribosomes extraits de micro-organismes comme facteur de croissance de culture de kératinocytes in vitro.

Par ailleurs, en étudiant l'activité des ribosomes de la présente demande, on a constaté que ceux-ci présentaient une remarquable activité inductrice "like" de l'EGF (Epidermal Growth Factor) facteur de croissance épidermique, sans présenter la toxicité de ce dernier. De ce fait les ribosomes peuvent être utilisés pour faciliter la régénération de la peau et de l'épiderme.

Ainsi les propriétés régénératrices cutanées par stimulation de la croissance cellulaire des compositions selon l'invention leur confèrent une efficacité dans la lutte contre le vieillissement de la peau.

La dose usuelle, variable selon le produit utilisé, le sujet traité et l'affection en cause, peut être, par exemple de 50 mcg à 5 mg par jour en applications locales chez l'homme.

L'invention a particulièrement pour objet les compositions cosmétiques ou dermatologiques telles que définies ci-dessus, caractérisées en ce qu'elles se présentent sous forme de préparations liquides ou solides à usage topique et notamment les compositions cosmétiques ou dermatologiques telles que définies ci-dessus caractérisées en ce qu'elles se présentent préférentiellement sous une des formes suivantes :

- des gels aqueux,
- des gels gras,
- des émulsions simples, eau dans huile,
- des émulsions simples, huile dans eau,
- des émulsions multiples, par exemple :
- eau dans huile dans eau ou huile dans eau dans huile,
- une émulsion triple eau dans huile dans eau,
- une émulsion triple huile dans eau dans huile,
- une émulsion huile dans eau contenant des cristaux liquides,
- des émulsions complexes contenant des cristaux liquides formant des bicouches lipidiques entourant des phases grasses,
- des pseudo-émulsions (dispersion d'une phase huileuse ou d'une émulsion eau dans huile dans une phase aqueuse gélifiée, sans tensio-actifs traditionnels),
- des micro-émulsions huile dans eau ou eau dans huile,
- des émulsions contenant deux phases huiles dispersées, différentes et insolubles entre elles,
- une pseudo-émulsion ou dispersion d'une phase grasse dispersée dans une phase aqueuse et stabilisée avec du Labragel (R), du Pemulen (R), de l'Hypan (R), de la gomme xanthane, de la CMC, de l'hydroxyéthyl cellulose, de l'Amigel (R), de la Polyvinyl-pyrrolidone, de l'Amercell HM 1500 (R), ou un mélange de deux ou plusieurs de ces gélifiants.

Les compositions selon l'invention peuvent le cas échéant contenir des filtres, des écrans aux radiations solaires, des extraits vitaminés, des parfums, des conservateurs, des anti-oxydants, des colorants.

Les compositions dermatologiques peuvent être, par exemple, solides ou liquides et se présenter sous les formes dermatologiques couramment utilisées, comme par exemple, les crèmes, les gels, les pommades, les lotions, les laits pour la peau, les gouttes, les collyres, les aérosols, les shampoings ou sous forme de liposomes ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions selon l'invention peuvent renfermer le cas échéant en plus des ribosomes extraits de microorganismes, d'autres composés actifs ayant des propriétés cutanées particulières.

Ainsi, ces compositions selon l'invention peuvent contenir par exemple l'acétate d'oléyle. Ce produit a des propriétés anti-lipasique et prévient directement la formation de comédons et de points noir.

Les compositions ainsi obtenues renferment deux constituants qui par leur action combinée constituent un produit bien adapté aux peaux à tendance acnéique.

Ces compositions selon l'invention peuvent aussi renfermer le cas échéant un composé favorisant la cicatrisation des lésions de l'acné, tel que par exemple, un extrait de Centella Asiatica et plus particulièrement un extrait glycolique ou un extrait sec titré de Centella Asiatica.

D'autres substances telles que l'huile d'onagre, les acides aminés peuvent aussi y être incorporées.

L'invention a donc plus particulièrement pour objet des compositions dermatologiques, caractérisées en ce qu'elles renferment des ribosomes extraits de microorganismes et le cas échéant un ou plusieurs des produits choisis parmi l'acétate d'oléyle, l'extrait sec titré de Centella Asiatica, l'huile d'onagre, les acides aminés.

Les compositions cosmétiques selon l'invention peuvent se présenter sous toutes les formes utilisées en cosmétologie : crème ou gel en pots ou en tubes, lait ou lotion en flacons de verre ou de matière plastique et éventuellement en flacon-doseur, ampoule, aérosol.

L'invention concerne donc des compositions cosmétiques, caractérisées en ce qu'elles se présentent sous forme de crème, gel, lait, lotion, émulsion, savon liquide, pain dermatologique, ou shampoing.

Pour chaque forme, on a recours à des excipients appropriés. Ces excipients doivent avoir toutes les qualités habituellement demandées. Ils doivent être doués d'une grande affinité pour la peau, être parfaitement bien tolérés, stables, présenter une consistance adéquate, permettant une utilisation facile et agréable.

A titre d'exemples d'excipients pouvant être utilisés, on peut citer notamment les hydrocarbures, les huiles de silicones, les triglycérides d'acide gras, les triglycérides de synthèse, les cires végétales, animales ou minérales, les acides et les alcools gras, les esters d'acides gras ou d'alcool gras, les amides d'acide gras, la lanoline, les tensio-actifs non ioniques, les tensio-actifs anioniques, les polysaccharides gélifiants naturels ou synthétiques, la chitine désacétylée, les dérivés de cellulose, les dérivés de guar, la polyols, les polyalkylèneglycols, les charges minérales, les pigments organiques ou les laques de colorants organiques ainsi que d'autres excipients connus et couramment utilisés tels que, par exemple, les polymères de type carboxyvinylique, les polyéthylèneglycols, le propylène glycol, des dérivés stéariques tel que, par exemple, le stéarate de glycérol, des alcools tels que, par exemple, les alcools stéaryliques, les alcools cétostéaryliques, l'alcool cétylique, l'éther cétylique polyoxyéthyléné, des huiles végétales telles que l'huile d'avocat, l'huile de tournesol, des huiles minérales telles que l'huile de vaseline, la glycérine, des dérivés de la lanoline, du talc, des mouillants, des épaississants, des stabilisants, des émulsionnants, des conservateurs, des parfums, des colorants.

Enfin des filtres solaires et des réflecteurs solaires peuvent être ajoutés à ces compositions cosmétiques pour leur conférer un pouvoir protecteur vis à vis des rayonnements solaires. Lorsque ces additifs sont insolubles dans les phases huiles et aqueuses, ils constituent donc une phase supplémentaire. Il seront choisis par exemple parmi les produits suivants :
- les perfluoroéthers comme les FOMBLIN (R) de la Société Montecatini,
- les pigments insolubles comme :
  . les oxydes de titane,
  . oxyde de titane rutile,
  . oxyde de titane anatase,
  . oxyde de titane pyrogéné comme le P 25 (R) de Degussa,
  . oxyde de titane micronisé dans le SUN VEIL (R) d'Ikeda,
  . oxyde de titane traité en surface par des silicones ou par des acides aminés, ou par de la lécithine, ou par des stéarates métalliques,
  . oxyde de fer,
  . oxyde de fer traité en surface par des silicones, ou par des acides aminés, ou par de la lécithine, ou par des stéarates métalliques,
  . oxyde de zinc,
  . oxyde zinc micronisé comme l'UFZO (R) de Cosmo Trends Corporation,
  . mica recouvert d'oxyde de titane.

Les différentes formes cosmétiques mentionnées ci-dessus peuvent être obtenues selon les méthodes usuelles utilisées dans ce domaine.

La présente invention concerne tout particulièrement les compositions cosmétiques ou dermatologiques telles que définies ci-dessus, caractérisées en ce que leurs excipients sont adaptés à l'application sur le visage, le cou et les mains.

La présente invention concerne également l'application à titre de produit cosmétique de compositions caractérisées en ce qu'elles renferment des ribosomes extraits de micro-organismes.

La présente invention concerne notamment une méthode de traitement cosmétique de régénération et de lutte contre le vieillissement de la peau caractérisée en ce que l'on applique sur la peau une quantité suffisante d'une composition cosmétique telle que définie ci-dessus.

Les exemples donnés ci-dessous illustrent l'invention sans toutefois la limiter.

## EXEMPLE 1 : Préparation de ribosomes

La technique utilisée dérive de celle décrite dans l'article référencé comme suit : JELENC P, Rapid purification of highly active ribosomes from Escherichia coli. Anal Biochem 1980 ; 105 : 369-74.

La souche utilisée est E. coli MRE600 de Microbiological Research Establishment, Salisbury, England, congelée et conservée à -20°C.

Les 3 tampons utilisés sont :

Tampon polymix I :

| | | |
|---|---|---|
| $MgCl_2$ | 5 mM | pH = 7,5 |
| $CaCl_2$ | 0,5 mM | |
| Putrescine | 8 mM | |
| Spermidine | 1 mM | |
| Tricine | 20 mM | |
| $NH_4Cl$ | 5 mM | |
| KCl | 95 mM | |
| DTT | 1 mM | |

Tampon polymix II :

| | | |
|---|---|---|
| $MgCl_2$ | 5 mM | pH = 7,5 |
| $CaCl_2$ | 0,5 mM | |
| Putrescine | 8 mM | |
| Spermidine | 1 mM | |
| Tricine | 20 mM | |
| $NH_4Cl$ | 150 mM | |
| KCl | 95 mM | |
| DTT | 1 mM | |

Tampon TRIS :

| | | |
|---|---|---|
| TRIS HCl | 20 mM | pH = 7,6 |
| $NH_4Cl$ | 60 mM | |
| $Mg(OAc)_2$ | 10 mM | |

Toutes les manipulations sont effectuées à 4°C.

- 127 g (poids humide) de bactéries E. coli sont décongelées et reprises dans 254 ml de tampon polymix I
- lyse des bactéries par trois passages à haute pression : V = 490 ml
- centrifugation : 30 minutes à 13000 rpm : V = 400 ml
- centrifugation : 30 minutes à 13000 rpm : V = 370 ml, $DO_{260}$ = 112480
- précipitation des protéines par sulfate d'ammonium (210 mg/ml) ; ajustement du pH à 7,5
- agitation 30 minutes, V = 395 ml
- centrifugation 30 minutes à 13000 rpm, $DO_{260}$ = 82855
- surnageant déposé sur SEPHACRYL S 200, prééquilibrage et élution par tampon Polymix II
- les fractions qui contiennent les ribosomes sont précipitées par le PEG 6000 (100 mg/ml)
- agitation 30 minutes
- centrifugation 15 minutes à 10.000 rpm
- dialyse une nuit contre 5 litres de tampon Tris.

**Expression des résultats et méthodes de calcul**

Une unité $A_{260}$ (absorption à 260 nm) correspond respectivement à 24 pmoles/ml de ribosomes 70S, 69 pmoles/ml de sous-unité 30S et 35 pmoles/ml de sous-unité 50S.

On obtient ainsi V = 44 ml, $DO_{260}$ = 7620 soit une suspension contenant 2,4 g de ribosomes 70S qui, si nécessaire, subit une filtration stérilisante et est ensuite répartie en aliquotes, congelés dans l'azote liquide, puis conservés à -80°C.

**EXEMPLE 2 : crème pour le visage**

- suspension de ribosomes    0,5 g
- acétate d'oléyle    2,0 g
- alcoyle phosphate de potassium    2,0 g
- palmitate d'éthyl hexyle    8,0 g
- lanoline hydrogénée    5,0 g
- triglycérides d'acides gras    4,0 g
- stéarate de sorbitan    1,0 g
- polymère carboxyvinylique neutralisé    0,4 g
- conservateurs    0,4 g
- composition aromatique    0,4 g
- eau purifiée q.s.p.    100 g

**EXEMPLE 3 : gel pour le visage**

- suspension de ribosomes    0,5 g
- extrait glycolique Centella Asiatica    5,0 g
- propylène glycol    5,0 g
- polymère carboxyvinylique neutralisé    0,8 g
- conservateurs    0,35 g
- composition aromatique    0,1 g
- eau purifiée q.s.p.    100 g

**EXEMPLE 4 : crème pour le corps**

- suspension de ribosomes    0,2 g
- stéarate de glycérol    4,0 g
- palmitate de sorbitan    6,0 g
- perhydrosqualène    5,0 g
- diisopropyle-cyclohexane    7,0 g
- triglycérides d'acides gras    9,0 g
- glycérine    5,0 g
- conservateurs    0,35 g
- composition aromatique    1,0 g
- eau purifiée q.s.p.    100 g

### EXEMPLE 5 : lotion tonique pour le visage

- suspension de ribosomes     0,05 g
- propylène glycol     5,0 g
- conservateurs     0,3 g
- composition aromatique     0,1 g
- alcool éthylique     10,0 g
- eau purifiée q.s.p.     100 ml

### EXEMPLE 6 : lait solaire

- suspension de ribosomes     0,1 g
- filtres solaires     5,0 g
- huile de vaseline     10,0 g
- cétéaryl octanoate     9,0 g
- huile de silicone     2,5 g
- éther cétylique P.O.E.     2,0 g
- stéarate de sorbitan     1,0 g
- conservateurs     0,35 g
- composition aromatique     0,5 g
- eau purifiée q.s.p.     100 ml

### EXEMPLE 7 : émulsion multiple

On chauffe à 80°C la phase aqueuse suivante, dite phase aqueuse interne :
- eau déminéralisée     26,52 g
- méthylparaben     0,1 g
- sulfate de magnésium     0,28 g
- glycérine 30°B     0,8 g
- O-cymen-5-ol     0,04 g

On chauffe séparément la phase grasse suivante :
- glycéryl isostéarate     2 g
- huile de ricin hydrogénée polyoxyéthylénée (7 moles)     0,2 g
- triglycérides capriques (capryliques)     8,2 g
- propylparaben     0,06 g
- huile de silicone volatile     1,6 g

On disperse la phase aqueuse dans la phase grasse à 80°C en agitant vigoureusement 5 minutes. Puis on refroidit lentement jusqu'à 25°C.

On disperse ensuite cette émulsion primaire eau/huile dans la phase aqueuse suivante, dite phase aqueuse externe, en mélangeant doucement à température ambiante :
- eau déminéralisée     QS 100 g
- lubragel MS (R)     15 g
- carbopol 980 (R)     0,03 g
- tétrasodium EDTA     0,054 g
- méthylparaben     0,216 g
- imidazolidinyl urée     0,216 g
- hydroxyde de sodium pur     0,125 g
- suspension de ribosomes     0,5 g

### EXEMPLE 8 : émulsion à phase gémellaires

On fait chauffer la phase grasse suivante à 80°C
- alcool stéarylique     1,0 g
- alcool cétylique     2,0 g
- cétéaryl octanoate     4,0 g
- polysorbate 60     4,0 g
- sorbitan stéarate     4,0 g
- caprylic caprique triglycérides     3,0 g

- beurre de karité        3,0 g
- acétate d'oleyle        2,0 g
- huile de silicone        0,5 g
- tocophérols        0,05 g

On fait chauffer la phase aqueuse suivante à 80°C
- eau déminéralisée        QS 100 g
- polymère carboxy vinylique        0,3 g
- conservateur        0,7 g
- lubragel MS (R)        5 g
- hydroxyde de sodium pur        0,3 g

On disperse la phase grasse dans la phase aqueuse et on agite vigoureusement pendant 10 minutes. On refroidit ensuite lentement l'émulsion ainsi formée jusqu'à 25°C, on y ajoute alors sous agitation modérée les ingrédients suivants :
- suspension de ribosomes        0,05 g
- parfum        0,2 g

**EXEMPLE 9** : **émulsion eau/silicone**

On chauffe à 60°C la phase grasse suivante :
- eau déminéralisée        73,12 g
- chlorure de sodium        0,8 g
- acide citrique pur        0,01 g
- méthylparaben        0,25 g
- propylène glycol        2 g
- 0-cymen-5-ol        0,1 g

On chauffe à 60°C la phase silicone suivante :
- stéarate d'isocétyle        3 g
- arlacel 83 (R)        0,8 g
- huile de ricin hydrogénée        0,3 g
- elfacos ST9 (R)        2,0 g
- acétate d'oleyle (anti-lipase)        0,15 g
- silicone DC 3225 (R) (DOW CORNING)        9,0 g
- silicone volatile        4,0 g

On disperse la phase aqueuse dans la phase silicone sous agitation moyenne pendant 10 minutes. On refroidit ensuite l'émulsion ainsi formée jusqu'à 25°C et on ajoute sous agitation faible :
- mélange de :
. suspension de ribosomes        1 g
. parfum        0,3 g

**EXEMPLE 10** : **émulsion sans émulsionnant**

On chauffe à 80°C la phase grasse suivante :
- huile de jojoba        4,0 g
- polyisobutène        4,0 g
- octyl stéarate        4,0 g
- acétate d'oleyle        2,0 g

On chauffe à 80°C la phase aqueuse suivante :
- glycérine 30° codex        3,0 g
- polymère carboxyvinylique        0,45 g
- lubrajel MS (R)        4,0 g
- hydroxyde de sodium pur        0,055 g
- conservateurs        0,55 g
- parfum        0,20 g
- eau déminéralisée        30,0 g

On disperse la phase grasse dans la phase aqueuse sous une très faible agitation et cisaillement élevé pendant 1/2 heure. On refroidit ensuite lentement l'émulsion ainsi formée jusqu'à 45°C, on ajoute alors sous bonne agitation :
- talc        3 g

Quand la dispersion du talc est bien terminée, on continue à refroidir sous agitation lente. Quand la température est de 25°C, on ajoute sous agitation modérée :
- eau déminéralisée      30 g
- suspension de ribosomes      0,05 g
préalablement mélangés.
Puis on ajoute, sous la même agitation :
- parfum      0,2 g

**EXEMPLE 11 :**

On prépare une émulsion huile dans eau de la manière suivante :
On chauffe à 80°C les composants de la phase grasse suivante :
- stéarate de glycérol autoémulsionnable (arlacel 165 (R) de la Sté ICI)      6 g
- alcool cétylique      1 g
- stérol de soja éthoxylé (générol 122 E 10 (R) de la Sté Henkel)      2 g
- mélange d'huile de vaseline et d'alcool de lanoline (amerchol L101 de la Sté Amerchol (R))      3 g
- pétrolatum et alcool de lanoline (amerchol CAB (R) de la Sté Amerchol)      1 g
- huile de carthame      6 g
- beurre de karité      3 g
- propyl paraben      0,05 g
Par ailleurs on prépare la phase aqueuse suivante que l'on porte également à 80°C :
- eau déminéralisée      60 g
- sorbitol à 70 %      3 g
- gomme xanthane      0,3 g
- méthylparaben      0,1 g
Quand la gomme xanthane est bien dispersée, on ajoute la phase grasse à la phase aqueuse à 80°C, et on agite vivement pendant 20 minutes. L'émulsion se forme.
Ensuite on réduit l'agitation et on refroidit lentement l'émulsion jusqu'à 40°C.
On y ajoute alors 2 g d'eau contenant 0,15 g d'imidazolidinyl urée, puis 0,3 g de parfum. A cette température on ajoutera 0,5 g de suspension de ribosomes.

**Etude de l'évaluation de l'action "stimulante" et "régénérante" des ribosomes selon l'invention sur des kératinocytes d'épiderme humain.**

Les méthodes in vitro permettent d'évaluer rapidement l'activité de produits de synthèse ou d'origine naturelle : ceux-ci sont directement mis en contact avec leur cible cosmétique, les cellules du derme ou de l'épiderme.

a) Matériels

L'actif cosmétique étudié est constitué par le produit de l'exemple 1 : la suspension de ribosomes.
La leucine (marquage au $^{14}C$ de tous les carbones, activité spécifique 11,7 GBq/mmole), et la thymidine (marquage au tritium sur le carbone en position 6, activité spécifique 1,07 TBq/mmole) ont été fournis par Amersham.
Tous les réactifs, milieux et additifs de culture ont été fournis par des sociétés commerciales telles que MERCK et GIBCO.
MCA = Milieu de culture pour l'attachement des kératinocytes, milieu MEM/199 (75/25 % v/v ; GIBCO) additionné de 50 UI/ml de pénicilline, de 50 µg/ml de steptomycine, de 10 % de sérum de veau foetal, de 10 ng/ml de toxine cholérique, de 5 µg/ml d'insuline bovine, de 0,4 µg/ml d'hydrocortisone, de 5 µg/ml de choline et de 8,5 µg/ml d'inositol.
MCI = Milieu de culture pour l'incubation en présence des composés soit milieu MCA sans EGF ni sérum de veau foetal (SVF).
Milieu utilisé pour le marquage des protéines par la $^{14}C$ leucine et de l'ADN par la $^{3}H$ thymidine : milieu MEM sans leucine (BIOPREDIC) additionné de 50 UI/ml de pénicilline, de 50 µg/ml de streptomycine, de 6,3 $10^{-7}$ M de leucine radiomarquée (7,4 kBq/ml) et de 3,4 $10^{-8}$ M de thymidine tritiée (37 kBq/ml).

b) Méthode expérimentale

L'actif cosmétique a été filtré puis rajouté dans le milieu MCI aux concentrations de 0,01 % et de 0,1 % (v/v).

Le prélèvement de peau a été effectué selon les recommandations du Comité National d'Ethique sur une femme de 73 ans ("post mortem").

Les kératinocytes ont été obtenus par dissociation à la trypsine de l'épiderme.

Les cellules, d'origine mammaire, ont été utilisées au 3ème passage. Elles ont été ensemencées dans des plaques de culture à 96 puits, à raison de 7 10⁴ cellules par puits dans 0,1 ml de milieu d'attachement (MCA). Elles ont été cultivées pendant 1 jour et utilisées avant confluence.

L'actif cosmétique a été mis en présence des cellules pendant 20 heures. Chaque condition expérimentale a été réalisée en triplicate. Des cultures témoins ont été effectuées en parallèle, en l'absence du produit à l'essai, soit dans le milieu MCI (culture témoin), soit dans le milieu MCA (culture témoin positif).

Pour l'incorporation de leucine et de thymidine radio-marquées, les kératinocytes ont été incubés avec la leucine radiomarquée au $^{14}$C et la thymidine tritiée pendant 4 heures.

Les tapis cellulaires ont été lavés afin d'éliminer les précurseurs non incorporés puis comptés en scintillation liquide. Les valeurs ont été exprimées en dpm/puits de culture.

c) Résultats :

Les essais ont été réalisés à deux concentrations de l'actif, 0,01 à 0,1 % (v/v), dans des conditions conventionnelles, c'est-à-dire dans des cultures effectuées sur un support plastique plan.

Les effets du composé ont été évalués par la mesure de deux paramètres fonctionnels, la néosynthèse des protéines et la néosynthèse d'ADN, après 20 heures d'incubation.

Le milieu d'incubation était dépourvu de sérum de veau foetal (SVF) et de facteur de croissance épidermique (EGF) pour faciliter la mise en évidence d'une stimulation cellulaire.

L'effet de l'addition de SVF et d'EGF dans le milieu de culture (témoin positif) a été étudié en parallèle.

Néosynthèse des protéines

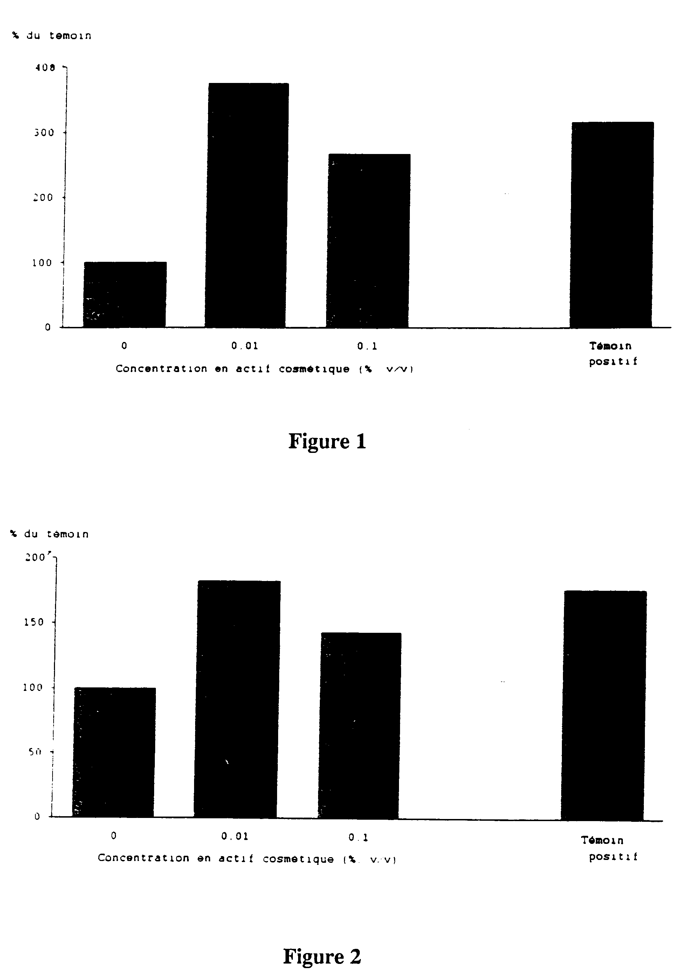

L'actif cosmétique augmente significativement ce paramètre aux deux concentrations étudiées, 0,01 et 0,1 % (v/v). L'effet maximal, + 274 % par rapport aux cultures témoins, est obtenu à la plus faible concentration ; il était comparable à celui constaté avec le témoin positif (tableau 1, figure 1).

Incorporation de la thymidine dans l'ADN

L'actif cosmétique augmente significativement ce paramètre aux deux concentrations étudiées, 0,01 et 0,1 % (v/v). L'effet maximal, +82 % par rapport aux cultures témoins, était obtenu à la plus faible concentration ; il était similaire à celui observé pour le témoin positif (tableau 1, figure 2).

Ces résultats montrent donc que dans les conditions expérimentales retenues, l'actif cosmétique a exercé un effet stimulant significatif vis-à-vis des kératinocytes humains, cette action cytostimulante étant maximale à la plus faible concentration essayée, 0,01 % (v/v).

TABLEAU 1 :

| Incorporation de ($^{14}$C)-leucine dans les protéines et de ($^{3}$H)-thymidine dans l'ADN de kératinocytes en culture incubés pendant 20 heures en présence de l'actif cosmétique | | | | |
|---|---|---|---|---|
| Paramètre | Actif cosmétique Concentration (%, v/v) | | | Témoin positif |
| | 0 | 0,01 | 0,1 | |
| Néosynthèse des protéines | 1125 1107 1396 **1209 +/- 162** (100) | 4726 4391 4450 **4522\* +/- 179** (374) | 3804 2743 3111 **3219\* +/- 539** (266) | 4167 3721 3526 **3805\* +/- 329** (315) |
| Néosynthèse d'ADN | 1254 1243 1504 **1334 +/- 148** (100) | 2689 2043 2561 **2431\* +/- 342** (182) | 1993 1846 1869 **1903\* +/- 79** (143) | 2253 2274 2528 **2352\* +/- 153** (176) |

Les résultats sont exprimés en dpm/puits de culture.

En caractères gras : moyenne et écart-type.

Entre ( ) : pourcentage par rapport aux cultures témoins.

* Moyenne des groupes différents du groupe témoin (p < 0,05).

**Revendications**

1) Compositions cosmétiques ou dermatologiques pour les soins de la peau, caractérisées en ce qu'elles renferment des ribosomes extraits de cellules végétales, animales ou de micro-organismes.

2) Compositions telles que définies à la revendication 1, caractérisées en ce que les ribosomes sont extraits de microorganismes eucaryotes ou procaryotes, de bactéries Gram⁻ choisies dans le groupe formé par Klebsiella pneumoniae, Hafnia, Escherichia coli, Klebsiella ozoenae, Enterobacter chloacae, Pseudomonas aeruginosa et Proteus ou de bactéries Gram$^+$ telles que par exemple le Bacillus subtilis, Arthrobacter sp., Lactobacillus ou le Staphylococcus tel que le Staphylococcus aureus.

**3)** Compositions telles que définies aux revendications 1 et 2, caractérisées en ce que les ribosomes sont extraits de bactéries Gram$^-$ choisies dans le groupe formé par Klebsiella pneumoniae, Hafnia, Escherichia coli.

**4)** Compositions telles que définies aux revendications 1 à 3, caractérisées en ce que les ribosomes sont extraits de la souche Klebsiella pneumoniae déposée à l'Institut Pasteur à Paris sous le numéro I-163.

**5)** Compositions telles que définies aux revendications 1 à 3, caractérisées en ce que les ribosomes sont extraits de la souche d'Hafnia déposée à l'Institut Pasteur à Paris sous le numéro I-868.

**6)** Compositions telles que définies aux revendications 1 à 3, caractérisées en ce que les ribosomes sont extraits de la souche d'Escherichia coli, déposée à l'Institut Pasteur à Paris sous le numéro I-870 ou de la souche MRE600 du Microbiological Research Establishment, Salisbury, England.

**7)** Compositions telles que définies aux revendications 1 à 6, caractérisées en ce qu'elles renferment $5.10^{-5}$ % à 1 % en poids d'une suspension de ribosomes extraits de micro-organismes ou cellules d'organisme supérieur.

**8)** Compositions telles que définies aux revendications 1 à 7, caractérisées en ce qu'elles renferment 0,005 % à 0,05 % en poids d'une suspension de ribosomes extraits de micro-organismes.

**9)** Compositions telles que définies aux revendications 1 à 8, caractérisées en ce qu'elles renferment 0,01 % en poids d'une suspension de ribosomes extraits de micro-organismes.

**10)** Compositions telles que définies aux revendications 1 à 9, caractérisées en ce que les ribosomes extraits de micro-organismes sont préparés selon les étapes successives suivantes :
- culture sur un milieu liquide ou solide de la souche de micro-organismes,
- récolte après complet développement des micro-organismes,
- lyse des micro-organismes,
  et traitement de ce lysat par centrifugation, précipitation au sulfate d'ammonium, centrifugation, tamisage moléculaire, précipitation au PEG 6000, centrifugation et dialyse de la solution obtenue.

**11)** Compositions telles que définies aux revendications 1 à 10, caractérisées en ce qu'elles présentent sous forme de préparations liquides ou solides à usage topique.

**12)** Compositions dermatologiques telles que définies aux revendications 1 à 11, caractérisées en ce qu'elles renferment des ribosomes extraits de microorganismes et le cas échéant un ou plusieurs des produits choisis parmi l'acétate d'oléyle, l'extrait sec titré de Centella Asatiaca, l'huile d'onagre, les acides aminés.

**13)** Compositions cosmétiques telles que définies aux revendications 1 à 11, caractérisées en ce qu'elles se présentent sous forme de crème, gel, lait, lotion, émulsion, savon liquide, pain dermatologique ou shampoing.

**14)** Compositions cosmétiques ou dermatologiques telles que définies aux revendications 1 à 11, caractérisées en ce que leurs excipients sont adaptés à l'application sur le visage, le cou et les mains.

**15)** Application à titre de produit cosmétique de compositions caractérisées en ce qu'elles renferment des ribosomes extraits de micro-organismes.

**16)** Méthode de traitement cosmétique de régénération et de lutte contre le vieillissement de la peau caractérisée en ce que l'on applique sur la peau une quantité suffisante d'une composition cosmétique telle que définie selon l'une quelconque des revendications 1 à 11.

**17)** Utilisation de ribosomes extraits de micro-organismes ou cellules d'organisme supérieur comme facteur de croissance cellulaire in vitro.

**18)** Utilisation de ribosomes extraits de micro-organismes comme facteur de croissance de culture de kératinocytes in vitro.

% du temoin

**Figure 1**

% du temoin

**Figure 2**

Office européen
des brevets

**RAPPORT DE RECHERCHE EUROPEENNE**

Numero de la demande

EP 94 40 1459

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | EP-A-0 297 457 (HEYL CHEMISCH-PHARMAZEUTISCHE FABRIK) * le document en entier * --- | 1-18 | A61K7/48 C12N5/08 |
| Y | DE-A-40 32 972 (ROUSSEL-UCLAF) * le document en entier * ----- | 1-18 | |

**DOMAINES TECHNIQUES RECHERCHES (Int.Cl.6)**

A61K
C12N

Le présent rapport a été établi pour toutes les revendications

| Lieu de la recherche | Date d'achèvement de la recherche | Examinateur |
|---|---|---|
| LA HAYE | 13 Octobre 1994 | Willekens, G |